(19) Europäisches Patentamt
European Patent Office
Office européen des brevets

(11) Publication number: **0 124 089**
**B1**

(12) # EUROPEAN PATENT SPECIFICATION

(45) Date of publication of patent specification: **04.03.87**

(51) Int. Cl.[4]: **C 07 D 213/85,** A 61 K 31/44

(21) Application number: **84104703.8**

(22) Date of filing: **26.04.84**

(54) **Novel 5-acyl-2-(1H)-pyridinones.**

(30) Priority: **29.04.83 US 490081**

(43) Date of publication of application:
**07.11.84 Bulletin 84/45**

(45) Publication of the grant of the patent:
**04.03.87 Bulletin 87/10**

(84) Designated Contracting States:
**AT BE CH DE FR GB IT LI LU NL SE**

(56) References cited:
**EP-A-0 089 022**
**US-A-4 083 849**

(73) Proprietor: **MERRELL DOW PHARMACEUTICALS INC.**
**2110 E. Galbraith Road**
**Cincinnati Ohio 45215 (US)**

(72) Inventor: **Jones, Winton D.**
**1464 Longacre Drive**
**Cincinnati, OH 45240 (US)**
Inventor: **Schnettler, Richard A.**
**9874 Forest Glen Drive**
**Cincinnati, OH 45242 (US)**
Inventor: **Dage, Richard C.**
**7825 Shadowhill Way**
**Cincinnati, OH 45242 (US)**

(74) Representative: **Vossius & Partner**
**Siebertstrasse 4 P.O. Box 86 07 67**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 124 089 B1

**Description**

Alkanoylpyridinones having the formula

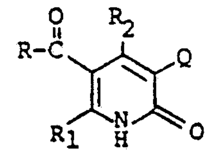

where Q is hydrogen or cyano, R and $R_1$ are each lower-alkyl and $R_2$ is hydrogen or methyl which are useful as cardiotonics are disclosed in EP—A—89 022 (published after the priority date of the present application).

This invention relates to certain 5-acyl-2-(1H)-pyridinones and to their use as cardiotonic agents.

More specifically, this invention relates to pharmaceutically active 5-acyl-2-(1H)-pyridinones of the formula (I)

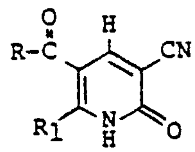

(I)

and the pharmaceutically acceptable salts thereof, wherein R is a straight chain $C_3$—$C_{10}$ alkyl group, and $R_1$ is methyl or ethyl.

These compounds are useful as cardiotonics in the treatment of cardiac failure and other conditions requiring strengthening of heart action with a cardiotonic agent.

Representative straight chain alkyl radicals are the n-propyl, n-butyl, pentyl, hexyl, septyl, octyl, nonyl and decyl group.

The compounds of formula I are useful both in the free base form and in the form of acid addition salts with both forms being within the purview of this invention. The acid addition salts are simply a more convenient form for use and, in practice, use of the salt amounts to use of the free base. The acids which can be used include those which produce, when combined with the free base, pharmaceutically acceptable salts, that is salts whose anions are relatively innocuous to the animal organism in pharmaceutical doses of the salts. In practice, it is convenient to form sulfate, phosphate, methansulfate or lactate salts. Others are those derived from mineral acids (e.g., hydrochloric), and organic acids such as acetic acid, citric acid, tartaric acid, ethanesulfonic acid, benzenesulfonic acid, *p*-toluenesulfonic acid and the like. The acid salts are prepared by standard techniques such as by dissolving the free base in aqueous or aqueous-alcohol solution or other suitable solvents containing the appropriate acid and isolating by evaporating the solution, or by reacting the free base and the acid in an organic solvent in which case the salt separates directly or can be obtained by concentration of the solution.

In general, the compounds of this invention are prepared by standard techniques analogously known in the art. A preferred synthesis for preparing the compounds of this invention conveniently involves the reaction of an appropriate 1-R-3-$R_1$-2-(dimethylamino-methylidenyl)-1,3-propanedione (II) with cyanacetamide (III) according to standard Michael addition reaction conditions. Preferably, the acetamide (III) is reacted with sodium hydride, under argon in an inert organic solvent, (e.g., tetrahydrofuran) to form an anion which is then condensed with the diketone (II) by heating the reactants together in an inert organic solvent, preferably tetrahydrofuran and the like. Preferably, the temperature of the reaction is about 50°C although the reaction proceeds well at temperatures between room temperature and 100°C. Heating is effected over a period of several hours although it is preferred to allow the reaction to proceed overnight. When R and $R_1$ are not the same a mixture of products are obtained which are separated quite nicely by flash chromatography wherein the reaction product mixture is admixed with 0.075—0.250 mm silica gel and the column is eluted with an appropriate solvent system (e.g., 35% ethylacetate — 65% methylene chloride). The fractions of eluate are monitored by thin layer chromatography.

The foregoing reaction is depicted as follows:

Reaction Scheme A:

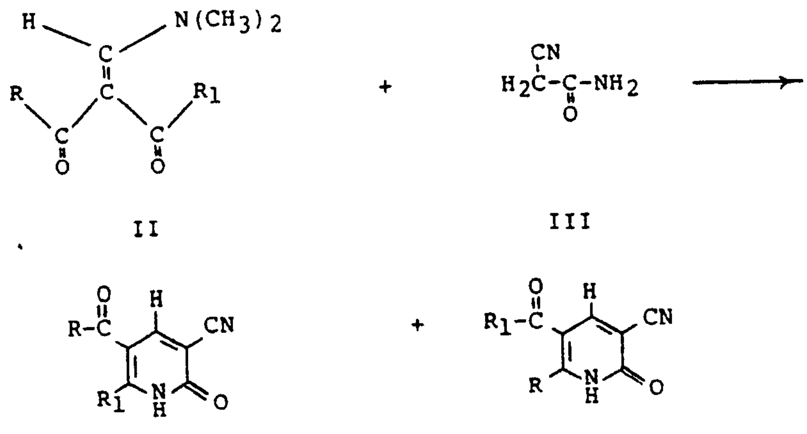

II III

IVA IVB

wherein R and $R_1$ are as previously defined.

The 1-R-3-$R_1$-2-[(dimethylamino)methylidenyl]-1,3-propanediones (III) are readily prepared by condensing the appropriately substituted 1,3-propandiones (V) with the appropriately substituted N,N-dimethylamino-dialkoxy methane (e.g., dimethylformamide acetals) according to standard condensation reaction conditions such as, for example, contacting equimolar quantities of the reactants together, optionally in an inert organic solvent and stirring the mixture for 1—12 hours at about room temperature. This reaction is depicted as follows:

Reaction Scheme B:

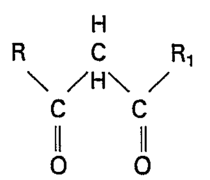 + $(CH_3O)_2CHN(CH_3)_2$→II

V VI

wherein R and $R_1$ are as previously defined.

## PREPARATION OF INTERMEDIATES

### Example 1

*4-[(Dimethylamino)methylenyl]-3,5-nonanedione*

A mixture of dimethylformamide dimethyl acetal (16.68 g, 0.136 mole) and 3,5-nonanedione (21.2 g, 0.136 mole) was stirred overnight at room temperature under argon. The resulting red oil was concentrated on the rotary evaporator to yield 4-[(Dimethylamino)methylenyl]-3,5-nonanedione.

### Example 2

*3-[(Dimethylamino)methylenyl]-2,4-octanedione*

A mixture of 2,4-octanedione (7.11 g, 0.50 mole) and dimethylformamide dimethylacetal (7.15 g, 0.60 mole) was stirred overnight at room temperature under argon. The resulting red oil was concentrated on the rotary evaporator then distilled on the kugelrohr at −15 mm, 140—155°C to yield 860 g (87%) of 3-[(dimethylamino)methylenyl]-2,4-octanedione.

### Example 3

*3-[(Dimethylamino)methylenyl]-2,4-nonanedione*

2,4-nonanedione (10.00 g, 0.0649 mole) and N,N-dimethylformamide dimethylacetal (8.97 g, 0.075 mole) were stirred overnight at room temperature under argon. The resulting orange liquid was concentrated on the rotary evaporator then distilled on the kugelrohr at −140°, 0.05 mm to yield 10.1 g (73%) of a light yellow liquid.

Example 4

*4-[(Dimethylamino)methylenyl]-3,5-decanedione*

3,5-Decanedione (10.00 g, 0.067 mole) and dimethylformamide dimethylacetal (8.97 g, 0.075 mole) were stirred at room temperature under argon overnight. The mixture was concentrated on the rotary evaporator to give the desired compound.

In a similar manner, by substituting the 1R-3-$R_1$-1,3-propanediones of the foregoing examples with the desired 1,3-propanediones bearing the desired R and $R_1$ moieties, and by substantially following the procedures of the foregoing examples, there are produced the desired intermediates necessary to prepare compounds (I) within the scope of this invention.

PREPARATION OF FINAL COMPOUNDS

Example 5

*5-(1-Oxopentyl)-1,2-dihydro-6-methyl-2-oxo-3-pyridine carbonitrile*

3-[(Dimethylamino)methylenyl]-2,4-octanedione (7.29 g, 0.037 mole) was added to a stirred suspension of cyanoacetamide (3.36 g, 0.04 mole) and sodium hydride (1.0 g, 0.04 mole). The reaction mixture was stirred and heated overnight at 50°C. The reaction mixture was neutralized to pH 6 with acetic acid and concentrated on the rotary evaporator. The residue was triturated with a 50:50 $CH_2Cl_2$—$H_2O$ mixture and collected. Approximately 3.0 g of this powder was mixed with 8.0 g of 0.075—0.250 mm silica gel and flash chromatographed eluting with 35% EtOAC—65% $CH_2Cl_2$ and collecting 65 ml fractions. 600 mg of 5-(1-oxopentyl)-1,2-dihydro-6-methyl-2-oxo-3-pyridine carbonitrile m.p. 216—217°C was collected in fractions 5 and 6. Th eflash chromatography described was continued to give 1.8 g of 5-acetyl-1,2-dihydro-6-butyl-2-oxo-3-pyridine carbonitrile m.p. 195—197°C in fractions 12 to 25, a compound not included within the scope of this invention.

Example 6

*5-(1-Oxopentyl)-1,2-dihdyro-6-ethyl-2-oxo-3-pyridine carbonitrile (II)*

4-[(Dimethylamino)methylenyl]-3,5-nonanedione (7.8 g, 0.037 mole) was added to a stirred suspension of cyanoacetamide (3.36 g, 0.04 mole) and sodium hydride (1.0 g, 0.04 mole). The reaction mixture was stirred and heated overnight at 50°C. The reaction mixture was neutralized to pH 6 with acetic acid and concentrated to dryness. The residue was triturated with a 50:50 $CH_2Cl_2$—$H_2O$ mixture to give a solid which was chromatographed over silica gel to give the title compound.

Example 7

*5-(1-Oxohexyl)-6-methyl-2-oxo-1,2-dihydro-3-pyridine carbonitrile*

3-[(Dimethylamino)methylenyl]-2,4-nonanedione (10.1 g, 0.475 mole) dissolved in dry THF (20 ml) was added all at once to a suspension of cyanoacetamide (4.20 g, 0.050 mole) and sodium hydride (2.5 g, 0.05 mole) in dry THF (175 ml) at room temperature under an argon atmosphere. The reaction mixture was than warmed to 50°C and stirred overnight. The reaction mixture was cooled to room temperature and neutralized with glacial acetic acid (5.5 ml). The resulting viscous orange mixture was concentrated *in vacuo* then partitioned between methylene chloride and water. The methylene chloride layer was separated, extracted with 5.0% sodium bicarbonate solution, separated, washed with brine, separated, dried ($MgSO_4$), filtered and concentrated on the rotary evaporator giving a solid. The solid was triturated with hexane (300 ml) and filtered to give 8.9 g (81%) m.p. 55—159° of a mixture of compound. Flash chromatography eluting with 15% EtOAC-hexane 85% gave the desired compound m.p. 178—180°C.

Example 8

*5-(1-Oxohexyl)-6-ethyl-2-oxo-1,2-dihydro-3-pyridine carbonitrile*

4-[(Dimethylamino)methylenyl]-3,5-decanedione (10.7 g, 0.0475 mole) dissolved in dry THF was added all at once to a suspension of cyanoacetamide (4.20 g, 0.05 mole) and sodium hydride (2.50 g, 0.05 mole) in dry THF at room temperature under argon. The reaction mixture was then stirred under argon at 50°C overnight. The reaction mixture was allowed to cool to room temperature and neutralized with glacial acetic acid. The resulting liquid was concentrated on the rotary evaporator then partitioned between methylene chloride and water. The methylene chloride layer was extracted with 5.0% $NaHCO_3$, washed with brine, separated, dried ($MgSO_4$) and concentrated. The residue was chromatographed giving the title compound.

In a similar manner, by substantially following the procedures set forth in the foregoing Examples (1—8), the compounds wherein R is an alkyl residue having more than 5 carbon atoms may readily be prepared. Such specific compounds include the 5-position heptanoyl, octanoyl, nonanoyl and decanoyl homologs of Examples 5-8.

The compounds of general Formula I may be used in the treatment of cardiac failure including congestive heart failure, backward heart failure, forward heart failure, left ventricular heart failure, or right ventricular heart failure or in the treatment of any other condition which requires the strengthening of heart action with a cardiotonic.

The utility of Formula I compounds as cardiotonics may be determined by administering the test compound (0.01—10 mg/kg) intravenously, intraperitoneally, intraduodenally or intragastrically in a suitable vehicle to a mongrel dog (either sex). The test dogs are anesthetized and prepared by isolating a suitable artery (e.g., femoral or common carotid) and vein (e.g., femoral or external jugular) introducing polyethylene catheters filled with 0.1% Heparin-Na to record arterial blood pressure and administer compounds, respectively. The chest is opened by splitting the sternum at the midline or by an incision at the left fifth intercostal space, and a pericardial cradle is formed to support the heart. A Walton-Brodie strain gage is sutured to the right or left ventricle to monitor myocardial contractile force. An electromagnetic flow probe may be placed around the root of the ascending aorta for measuring cardiac output less coronary blood flow. Heart failure is induced by administering sodium pentobarbital (20 to 40 mg/kg) followed by a continuous infusion of 1—2 mg/kg/min. or propranalol hydrochloride (4 mg/kg) followed by a continuous infusion of 0.18 mg/kg/min. to the blood perfusing the heart. Following administration of either of these cardiac depressants, the right aterial pressure dramatically increases and cardiac output is severely depressed. Reversal of these effects by the test compound indicates cardiotonic activity.

The compounds may be administered in various manners to achieve the desired effect. The compounds may be administered alone or in the form of pharmaceutical preparations to the patient being treated either orally or parenterally, that is, intravenously or intramuscularly. The amount of compound administered will vary with the patient, the severity of the cardiac failure and the mode of administration.

For oral or parenteral administration the cardiotonically effective amount of compound is from about 0.01 mg/kg of patients body weight per day up to about 500 mg/kg of patient body weight per day and preferably from about 0.10 mg/kg of patient body weight per day up to about 200 mg/kg of patient body weight per day.

For oral administration a unit dosage may contain, for example, from 1.0 to 750 mg of the active ingredient, preferably about 10 to 250 mg of the active ingredient. For parenteral administration a unit dosage may contain, for example, from 5 to 500 mg of the active ingredient, preferably about 10 to 250. Repetitive daily administration of the compounds may be desired and will vary with the condition of the patient and the mode of administration.

As used herein the term patient is taken to mean warm blooded animals, for example, birds, such as chickens and turkeys, and mammals, such as primates, humans, sheep, horses, bovine cows and bulls, pigs, dogs, cats, rats and mice.

For oral administration the compounds can be formulated into solid or liquid preparations such as capsules, pills, tablets, troches, powders, solutions, suspensions or emulsions. The solid unit dosage forms can be a capsule which can be of the ordinary gelatin type containing, for example, lubricants and inert filler, such as lactose, sucrose and cornstarch. In another embodiment the compounds of general Formula I can be tableted with conventional tablet bases such as lactose, sucrose and cornstarch in combination with binders, such as acacia, cornstarch or gelatin, disintegrating agents such as potato starch or alginic acid, and a lubricant such as stearic acid or magnesium stearate.

For parenteral administration the compounds may be administered as injectable dosages of a solution or suspension of the compound in a physiologically acceptable diluent with a pharmaceutical carrier which can be a sterile liquid such as water, alcohols, oils and other acceptable organic solvents with or without the addition of a surfactant and other pharmaceutically acceptable adjuvants. Illustrative of oils which can be employed in these preparations are those of petroleum, animal, vegetable, or synthetic origin, for example, peanut oil, soybean oil and mineral oil. In general, water, saline, aqueous dextrose and related sugar solutions, ethanol and glycols such as propylene glycol or polyethylene glycol or 2-pyrrolidone are preferred liquid carriers, particularly for injectable solutions.

The compounds can be administered in the form of a depot injection or implant preparation which may be formulated in such a manner as to permit a sustained release of the active ingredient. The active ingredient can be compressed into pellets or small cylinders and implanted subcutaneously or intramuscularly as depot injections or implants. Implants may employ inert materials such as biodegradable polymers or synthetic silicones, for example, Silastic, a silicone rubber manufactured by the Dow-Corning Corporation.

As is true in many large classes of compounds certain subgeneric members and certain specific members of the class are preferred for the pharmaceutical activity in treating disease states in man. In this instance the preferred compounds of formula I are those wherein R is n-butyl or n-pentyl. The most preferred specific compounds are 3-cyano-6-methyl-5-n-pentanoylp2(1H)pyridinone, and 3-cyano-6-ethyl-5-n-pentanoyl-2(1H)-pyridinone. Of the remaining members of the generic class of compounds the next preferred compounds are those wherein R is pentyl, i.e., the specific compounds 3-cyano-6-methyl-5-n-hexanoyl-2(1H)pyridinone and 3-cyano-6-ethyl-5-n-hexanoyl-2(1H)pyridinone.

**Claims for the Contracting States: BE CH DE FR GB IT LI LU NL SE**

1. A compound of the formula I

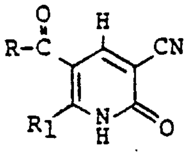

(I)

and the pharmaceutically acceptable salts thereof, wherein R is a straight chain $C_3$—$C_{10}$ alkyl group and $R_1$ is methyl or ethyl.

2. A compound of claim 1 wherein R is n-butyl.

3. A compound of claim 1 wherein R is n-pentyl.

4. A compound of claim 1 wherein $R_1$ is methyl.

5. A compound of claim 1 wherein $R_1$ is ethyl.

6. A compound of claim 1, said compound being 5-n-pentanoyl-1,2-dihydro-6-methyl-2-oxo-3-pyridine carbonitrile.

7. A compound of claim 1, said compound being 5-n-pentanoyl-1,2-dihydro-6-ethyl-2-oxo-3-pyridine carbonitrile.

8. A compound of claim 1, said compound being 5-n-hexanoyl-6-methyl-2-oxo-1,2-dihydro-3-pyridine carbonitrile.

9. A compound of claim 1, said compound being 5-n-hexanoyl-6-ethyl-2-oxo-1,2-dihydro-3-pyridine carbonitrile.

10. A compound of the formula I according to any of claims 1 to 9 and the pharmaceutically acceptable salts thereof for use in treating cardiac failure.

11. A process for preparing compounds of the formula I according to claim 1 which comprises reacting an appropriate 1-R-3-$R_1$-2-(dimethylamino-methylidenyl)-1,3-propanedione with cyanacetamide, and optionally converting the product obtained into a pharmaceutically acceptable salt.

12. A process of claim 11 wherein said reaction takes place under standard Michael addition reaction conditions.

13. A process of claim 12 wherein the 1,3-propanedione is 3-[(dimethylamino)methylenyl]-2,4-octanedione.

14. A process of claims 11—13 wherein the products produced are separated by flash chromatography.

15. A pharmaceutical composition comprising a compound according to any of claims 1 to 9 and a pharmaceutically acceptable carrier or diluent.

**Claims for the Contracting State: AT**

1. A process for preparing a compound of the formula I

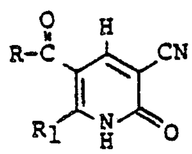

(I)

and the pharmaceutically acceptable salts thereof, wherein R is a straight chain $C_3$—$C_{10}$ alkyl group and $R_1$ is methyl or ethyl which comprises reacting an appropriate 1-R-3-$R_1$-2-(dimethylamino-methylidenyl)-1,3-propandione with cyanacetamide, and optionally converting the product obtained into a pharmaceutically acceptable salt.

2. The process of claim 1 for preparing a compound of formula (I) wherein R is n-butyl.

3. The process of claim 1 for preparing a compound of formula (I) wherein R is n-pentyl.

4. The process fo claim 1 for preparing a compound of formula (I) wherein $R_1$ is methyl.

5. The process of claim 1 for preparing a compound of formula (I) wherein $R_1$ is ethyl.

6. The process of claim 1 for preparing the compound 5-n-pentanoyl-1,2-dihydro-6-methyl-2-oxo-3-pyridine carbonitrile.

7. The process of claim 1 for preparing the compound 5-n-pentanoyl-1,2-dihydro-6-ethyl-2-oxo-3-pyridine carbonitrile.

8. The process of claim 1 for preparing the compound 5-n-hexanoyl-6-methyl-2-oxo-1,2-dihydro-3-pyridine carbonitrile.

9. The process of claim 1 for preparing the compound 5-n-hexanoyl-6-ethyl-2-oxo-1,2-dihydro-3-pyridine carbonitrile.

10. The process of claim 1 wherein said reaction takes place under standard Michael addition reaction conditions.

11. A process of claim 1 wherein the 1,3-propanedione is 3-[(dimethylamino)methylenyl]-2,4-octanedione.

12. The process of claim 1 wherein the products produced are separated by flash chromatography.

**Patentansprüche für die Vertagsstaaten: BE CH DE FR GB IT LI LU NL SE**

1. Eine Verbindung der Formel I

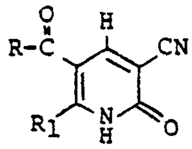

(I)

und ihre pharmazeutisch verträglichen Salze, in der R einen unverzweigten $C_3$—$C_{10}$-Alkylrest und $R_1$ eine Methyl- oder Äthylgruppe bedeuten.

2. Eine Verbindung nach Anspruch 1, in der R die n-Butylgruppe ist.

3. Eine Verbindung nach Anspruch 1, in der R die n-Pentylgruppe ist.

4. Eine Verbindung nach Anspruch 1, in der $R_1$ die Methylgruppe ist.

5. Eine Verbindung nach Anspruch 1, in der $R_1$ die Äthylgruppe ist.

6. Eine Verbindung nach Anspruch 1, nämlich 5-n-Pentanoyl-1,2-dihydro-6-methyl-2-oxo-3-pyridin-carbonitril.

7. Eine Verbindung nach Anspruch, nämlich 5-n-Pentanoyl-1,2-dihydro-6-äthyl-2-oxo-3-pyridin-carbonitril.

8. Eine Verbindung nach Anspruch 1, nämlich 5-n-Hexanoyl-6-methyl-2-oxo-1,2-dihydro-3-pyridin-carbonitril.

9. Eine Verbindung nach Anspruch 1, nämlich 5-n-Hexanoyl-6-äthyl-2-oxo-1,2-dihydro-3-pyridin-carbonitril.

10. Eine Verbindung der Formel I nach einem der Ansprüche 1 bis 9 und ihre pharmazeutisch verträglichen Salze zur Verwendung bei der Behandlung von Herzversagen.

11. Verfahren zur Herstellung von Verbindungen der Formel I nach Anspruch 1 durch Umsetzung eines entsprechenden 1-R-3-$R_1$-2-(Dimethylamino-methylidenyl)-1,3-propandions mit Cyanacetamid und gegebenenfalls Unwandlung des erhaltenen Produkts in ein Pharmazeutisch verträgliches Salz.

12. Verfahren nach Anspruch 11, wobei die Umsetzung unter den üblichen Bedingungen der Michael-Additionsreaktion durchgeführt wird.

13. Verfahren nach Anspruch 12, wobei das 1,3-Propandion 3-[(Dimethylamino)-methylenyl]-2,4-octandion ist.

14. Verfahren nach den Ansprüchen 11 bis 13, wobei die Produkte durch flash-Chromatographie getrennt werden.

15. Arzneimittel, enthaltend eine Verbindung nach einem der Ansprüche 1 bis 9 und einen pharmazeutisch verträglichen Träger oder ein Verdünnungsmittel.

**Patentansprüche für den Vertragsstaat: AT**

1. Verfahren zur Herstellung einer Verbindung der Formel I

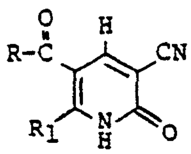

(I)

und ihrer pharmazeutisch verträglichen Salze, in der R einen unverzweigten $C_3$—$C_{10}$-Alkylrest und $R_1$ eine Methyl- oder Äthylgruppe bedeuten, durch Umsetzung eines geeigneten 1-R-3-$R_1$-2-(Dimethylamino-methylidenyl)-1,3-propandions mit Cyanacetamid und gegebenenfalls Umwandlung des erhaltenen Produkts in ein pharmazeutisch verträgliches Salz.

2. Verfahren nach Anspruch 1 zur Herstellung einer Verbindung der Formel I, in der R die n-Butylgruppe ist.

3. Verfahren nach Anspruch 1 zur Herstellung einer Verbindung der Formel I, in der R die n-Pentylgruppe ist.

4. Verfahren nach Anspruch 1 zur Herstellung einer Verbindung der Formel I, in der $R_1$ die Methylgruppe ist.

5. Verfahren nach Anspruch 1 zur Herstellung einer Verbindung der Formel I, in der $R_1$ die Äthylgruppe ist.

6. Verfahren nach Anspruch 1 zur Herstellung einer Verbindung 5-n-Pentanoyl-1,2-dihydro-6-methyl-2-oxo-3-pyridin-carbonitril.

7. Verfahren nach Anspruch 1 zur Herstellung einer Verbindung 5-n-Pentanoyl-1,2-dihydro-6-äthyl-2-oxo-3-pyridin-carbonitril.

8. Verfahren nach Anspruch 1 zur Herstellung einer Verbindung 5-n-Haxanoyl-6-methyl-2-oxo-1,2-dihydro-3-pyridin-carbonitril.

9. Verfahren nach Anspruch 1 zur Herstellung einer Verbindung 5-n-Haxanoyl-6-äthyl-2-oxo-1,2-dihydro-3-pyridin-carbonitril.

10. Verfahren nach Anspruch 1, wobei die Umsetzung unter den üblichen Bedingungen einer Michael-Additionsreaktion durchgeführt wird.

11. Verfahren nach Anspruch 1. wobei das 1,3-Propandion 1-[(Dimethylamino)-methylenyl)-2,4-octandion ist.

12. Verfahren nach Anspruch 1, wobei die erhaltenen Produkte durch Flash-Chromatophraphie getrennt werden.

**Revendications pour les Etats contractants: BE CH DE FR GB IT LI LU NL SE**

1. Un composé de formule I

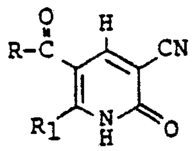

(I)

dans laquelle R représente un groupe alkyle à chaîne droite en $C_3$—$C_{10}$ et $R_1$ représente un groupe méthyle ou éthyle, et leurs sels acceptables pour l'usage pharmaceutique.

2. Un composé selon la revendication 1, dans lequel R représente un groupe n-butyle.

3. Un composé selon la revendication 1, dans lequel R représente un groupe n-pentyle.

4. Un composé selon la revendication 1, dans lequel $R_1$ représente un groupe méthyle.

5. Un composé selon la revendication 1, dans lequel $R_1$ représente un group éthyle.

6. Un composé selon la revendication 1, consistant en le 5-n-pentanoyl-1,2-dihydro-6-méthyl-2-oxo-3-pyridine-carbonitrile.

7. Un composé selon la revendication 1, consistant en le 5-n-pentanoyl-1,2-dihydro-6-éthyl-2-oxo-3-pyridine-carbonitrile.

8. Un composé selon la revendication 1, consistant en le 5-n-hexanoyl-6-méthyl-2-oxo-1,2-dihydro-3-pyridine-carbonitrile.

9. Un composé selon la revendication 1, consistant en le 5-n-hexanoyl-6-éthyl-2-oxo-1,2-dihydro-3-pyridine-carbonitrile.

10. Un composé de formule I selon l'une quelconque des revendications 1 à 9 et ses sels acceptables pour l'usage pharmaceutique pour l'utilisation dans le traitements des troubles cardiaques.

11. Un procédé pour préparer les composés de formule I selon la revendication 1, qui consiste à faire réagir une 1-R-3-$R_1$-2-(diméthylamino-méthylidényl)-1,3-propanedione appropriée avec le cyanacétamide et facultativement à convertir le produit obtenu en un sel acceptable pour l'usage pharmaceutique.

12. Un procédé selon la revendication 11, dans lequel la réaction est exécutée dans les conditions classiques pour une réaction d'addition de Michael.

13. Un procédé selon la revendication 12, dans lequel la 1,3-propanedione est la 3-[(diméthyl-amino)méthylényl]-2,4-octanedione.

14. Un procédé selon les revendications 11 à 13, dans lequel les produits obtenus sont séparés par chromatographie rapide.

15. Une composition pharmaceutique comprenant un composé selon l'une quelconque des revendications 1 à 9 et un véhicule ou diluant acceptable pour l'usage pharmaceutique.

**Revendications pour l'Etat contractant: AT**

1. Un procédé de préparation d'un composé de formule I

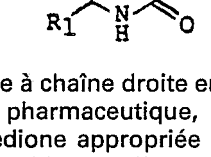

(I)

dans laquelle R représente un groupe alkyle à chaîne droite en $C_3$—$C_{10}$ et $R_1$ un groupe méthyle ou éthyle, et de ses sels acceptables pour l'usage pharmaceutique, qui consiste à faire réagir une 1-R-3-$R_1$-2-(diméthylamino-méthylidényl)-1,3-propanedione appropriée avec le cyanacétamide et facultativement à convertir le produit obtenu en un sel acceptable pour l'usage pharmaceutique.

2. Le procédé de la revendication 1, pour la préparation d'un composé de formule I dans laquelle R représente un groupe n-butyle.

3. Le procédé de la revendication 1, pour préparer un composé de formula I dans laquelle R représente un groupes n-pentyle.

4. Le procédé de la revendication 1, pour préparer un composé de formula I dans laquelle $R_1$ représente un groupe méthyle.

5. Le procédé de la revendication 1, pour préparer un composé de formula I dans laquelle $R_1$ représente un groupe éthyle.

6. Le procédé de la revendication 1, pour préparer le composé: 5-n-pentanoyl-1,2-dihydro-6-méthyl-2-oxo-3-pyridine-carbonitrile.

7. Le procédé de la revendication 1, pour préparer le composé: 5-n-pentanoyl-1,2-dihydro-6-éthyl-2-oxo-3-pyridine-carbonitrile.

8. Le procédé de la revendication 1, pour préparer le composé: 5-n-hexanoyl-6-méthyl-2-oxo-1,2-dihydro-3-pyridine-carbonitrile.

9. Le procédé de la revendication 1, pour préparer le composé: 5-n-hexanoyl-6-éthyl-2-oxo-1,2-dihydro-3-pyridine-carbonitrile.

10. Le procédé de la revendication 1, dans lequel la réaction est effectuée dans les conditions classiques pour une réaction d'addition de Michael.

11. Un procédé selon la revendication 1, dans lequel la 1,3-propanedione est la 3-[(diméthylamino)méthylényl]-2,4-octanedione.

12. Le procédé de la revendication 1, dans lequel les produits obtenus sont séparés par chromatographie rapide.